# EUROPÄISCHE PATENTSCHRIFT

(11) **EP 2 681 072 B1**
(45) Veröffentlichungstag und Bekanntmachung des Hinweises auf die Patenterteilung: **20.01.2016**
(21) Anmeldenummer: 12705065.6
(22) Anmeldetag: 17.02.2012
(51) Int. Cl.: B60N 2/22, B60N 2/20, B60N 2/68, B60N 2/225, B60N 2/235

(54) **BESCHLAG FÜR EINEN FAHRZEUGSITZ**
FITTING FOR VEHICLE SEAT
FERRURE POUR SIÈGE DE VÉHICULE

(30) Priorität: 04.03.2011 DE 102011013163
(43) Veröffentlichungstag der Anmeldung: 08.01.2014
(73) Patentinhaber: Johnson Controls Components GmbH & Co. KG, 67657 Kaiserslautern (DE)
(72) Erfinder: ASSMANN, Uwe, 42855 Remscheid (DE)
(74) Vertreter: Liedhegener, Ralf
(86) Internationale Anmeldenummer: PCT/EP2012/000689
(87) Internationale Veröffentlichungsnummer: WO 2012/119698

(56) Entgegenhaltungen:
- DE-A1-102008 024 052
- DE-B4-102004 010 491
- FR-A1- 2 578 602
- FR-A1- 2 900 605

## Beschreibung

Die Erfindung betrifft einen Beschlag für einen Fahrzeugsitz, insbesondere für einen Kraftfahrzeugsitz, mit den Merkmalen des Oberbegriffs des Anspruches 1.

Aus der EP 1 676 502 A2 ist ein Beschlag bekannt, der eine direkte Lagerung der metallischen Beschlagteile aneinander in der Nähe des Außenumfangs des zweiten Beschlagteil vorsieht, wobei der Umklammerungsring L-förmig ausgebildet ist, fest mit dem ersten Beschlagteil am Außenumfang desselben oder in nächster Nähe hiervon verbunden ist, einen zylinderförmigen Bereich und einen sich nach innen erstreckenden Randbereich aufweist, wobei er mit dem sich nach innen erstreckenden Randbereich den Außenumfangsbereich des zweiten Beschlagteils hintergreift und mit einer Mehrzahl in axialer Richtung in Richtung des ersten Beschlagteils vorstehender Vorsprünge in Gleitkontakt mit einer nach außen weisenden Seitenfläche des ersten Beschlagteils ist. Die Lagerung in radialer Richtung kann entweder über den Umklammerungsring oder - alternativ - über das erste Beschlagteil, das einen entsprechenden Absatz in der Nähe des Außenumfangs aufweist, erfolgen. Hierbei ergibt sich ein ungünstiger Reibungskoeffizient der Materialpaarung Metall-Metall.

Aus der DE 10 2004 010 491 B4 ist ein Beschlag für einen Fahrzeugsitz bekannt, von dem ein Schnitt in der Zeichnung dargestellt ist. Dieser Beschlag weist ein erstes Beschlagteil 111, ein auf dem ersten Beschlagteil 111 gelagertes und relativ zu diesem um eine zentrale Achse drehbares zweites Beschlagteil 112, und einen Umklammerungsring 121 auf, welcher mit dem ersten Beschlagteil 111 verbunden ist und das zweite Beschlagteil zum axialen Sichern übergreift. Hierbei ist zwischen dem L-förmigen Umklammerungsring 121 und dem zweiten Beschlagteil 112 ein Zwischenring 131 aus Kunststoff angeordnet, welcher L-förmig ausgebildet ist, und entsprechend dem L-förmigen Umklammerungsring 121 verlaufend an dessen Innenmantelfläche und innerer Seitenfläche positioniert ist. Der Zwischenring 131 ist drehfest mit dem zweiten Beschlagteil 112 verbunden und lagert das erste Beschlagteil 111 in radialer Richtung mit einer durch die Innenmantelfläche des zylinderförmigen Bereichs gebildeten Lagerfläche und in axialer Richtung durch die in radialer Richtung verlaufende Stirnfläche.

Die FR 2 900 605 A1 (dem Oberbegriff entsprechend) offenbart einen Beschlag mit zwei Beschlagteilen und einem im Wesentlichen Z-förmigen Umklammerungsring, der während der Montage des Beschlages mit einem Spanndruck beaufschlagt wird. Durch den Spanndruck verschieben sich einzelne Segmente des Umklammerungsrings solange relativ zu einem ersten Beschlagteil, bis ein in radialer Richtung nach innen gezogener Rand des Umklammerungsrings auf einer Lagerfläche eines zweiten Beschlagteils aufsitzt. Anschließend wird der Umklammerungsring an das erste Beschlagteil gefügt und dadurch die Ausrichtung zwischen Umklammerungsring und erstem Beschlagteil fixiert. Nachteilig an dieser ist, dass sich der Umklammerungsring während der Montage des Beschlages in radialer Richtung relativ zu dem ersten Beschlagteil verschieben lassen muss. Dies bedingt eine Kontaktfläche zwischen dem ersten Beschlagteil und dem zweiten Beschlagteil in einer radial nach außen verlaufenden Ebene, welche als Gleitebene zwischen den beiden gepaarten Bauteilen dient. Diese Gleitebene stellt jedoch eine im Betrieb des Beschlages hochbelastete Ebene dar, so dass hohe Anforderungen an die Verbindung zwischen dem Umklammerungsring und dem ersten Beschlagteil in dieser Ebene zu stellen sind. Ein einfaches, kostengünstiges und formschlüssiges Umgreifen des ersten Beschlagteils durch einen hohlzylindrischen Bereich des Umklammerungsrings kann ebenfalls nicht erfolgen.

Aus der FR 2 578 602 A1 ist ein Beschlag mit zwei Beschlagteilen und einem komplex geformten, im Wesentlichen C-förmigen Umklammerungsring bekannt. Der Umklammerungsring umgreift ein erstes Beschlagteil außen formschlüssig und lagert in einer in Umfangsrichtung verlaufenden Fläche unter Zwischenlage eines Kugellagers das zweite Beschlagteil.

Der Erfindung liegt die Aufgabe zu Grunde, einen Beschlag der eingangs genannten Art zu verbessern. Diese Aufgabe wird erfindungsgemäß durch einen Beschlag mit den Merkmalen des Anspruches 1 gelöst. Vorteilhafte Ausgestaltungen sind Gegenstand der Unteransprüche.

Hierbei ist am zweiten Beschlagteil ein gegenüber dem maximalen Außendurchmesser nach innen versetzter Absatz mit sich in radialer Richtung nach außen erstreckender Stirnfläche und einer zylinderförmigen Lagerfläche ausgebildet, wobei die Lagerfläche einen Durchmesser hat, der kleiner als der maximale Außendurchmesser des zweiten Beschlagteils ist, und die zylinderförmige Lagerfläche den Rand des Umklammerungsrings direkt oder indirekt, insbesondere bevorzugt über einen drehfest mit dem Umklammerungsring verbundenen Zwischenring, lagert. Im Unterschied zum Stand der Technik erfolgt hierbei eine Verlagerung der Lagerfläche von einer Position am Außenumfang des zweiten Beschlagteils radial nach innen, in Richtung der zentralen Achse des Beschlags. Hierbei ragt zumindest ein Bereich mit maximalem Außendurchmesser des zweiten Beschlagteils über die am zweiten Beschlagteil ausgebildete Lagerfläche radial nach außen, wobei dieser Bereich im Crashfall eine Abstützung für den Umklammerungsring bilden kann und dadurch die Crashsicherheit erhöht. Im normalen Betrieb ist jedoch dieser Bereich nicht in Kontakt mit dem Umklammerungsring.

Insbesondere bevorzugt ist ein Zwischenring vorgesehen, der drehfest mit dem Umklammerungsring verbunden und an seiner Innenmantelfläche auf einem Absatz des zweiten Beschlagteils gelagert ist. Dadurch, dass zwischen dem Umklammerungsring und einem Absatz des zweiten Beschlagteils ein Zwischenring angeordnet ist, kann das zum Betätigen notwendige Spiel so ausgeglichen werden, dass auch ohne Lehnenkompensationsfeder keine störenden Klappergeräusche entstehen. Um den Verschleiß gering zu halten, d.h. auf wenige ausgewählte Stellen zu beschränken, wird der Zwischenring vom Umklammerungsring drehfest mitgenommen.

Der Umklammerungsring weist vorzugsweise Mittel zum drehfesten Halten des Zwischenrings auf. Diese Mittel können insbesondere durch Aufnahmen am Umklammerungsring und Nocken am Zwischenring und/oder durch Öffnungen am Umklammerungsring und Zapfen am Zwischenring gebildet sein. Es sind jedoch auch beliebige andere Mittel zum drehfesten Halten des Zwischenrings am Umklammerungsring möglich.

Der Zwischenring weist zum drehfesten Halten des Zwischenrings am Umklammerungsring vorzugsweise mindestens drei Nocken auf, insbesondere bevorzugt genau sechs, welche sich in radialer Richtung von einem im Wesentlichen hohlzylindrischen Bereich aus nach außen erstrecken. Diese Nocken werden beim Zusammenbau des Beschlags elastisch und/oder plastisch verformt. Der Umklammerungsring weist vorzugsweise mindestens eine der Anzahl der Nocken entsprechende Anzahl von Aussparungen auf, von denen die Nocken aufgenommen werden. Hierbei kann die Anzahl doppelt, dreifach oder vierfach so groß sein, wodurch sich die Zahl möglicher Ausrichtungen des Zwischenrings im Umklammerungsring vergrößert.

Der Zwischenring weist zum drehfesten Halten des Zwischenrings am Umklammerungsring vorzugsweise mindestens drei Zapfen auf, insbesondere bevorzugt genau sechs, welche sich in axialer Richtung vom sich in radialer Richtung erstreckenden Randbereich des Zwischenrings in Richtung Umklammerungsring erstrecken. Der Umklammerungsring weist mindestens eine entsprechende Anzahl von Öffnungen auf, in welchen die Zapfen beim Zusammenbau positioniert werden.

Insbesondere bevorzugt sind das zweite Beschlagteil und der Zwischenring mit Fasen versehen, um das Aufschieben des Zwischenrings auf den Absatz des zweiten Beschlagteils zu erleichtern. Dadurch werden auch Beschädigungen der Gleitfläche des Zwischenrings vermieden.

Der Zwischenring übt im zusammengebauten Zustand des Beschlags eine radiale Vorspannkraft auf das zweite Beschlagteil aus, so dass das zweite Beschlagteil relativ zum Umklammerungsring (und damit in Bezug auf das erste Beschlagteil) zentriert gehalten wird.

Bei ordnungsgemäßem Gebrauch ist ein radialer Spalt zwischen dem zweiten Beschlagteil und dem Umklammerungsring vorgesehen. Dahingegen kann im Crashfall eine zusätzliche direkte Abstützung des zweiten Beschlagteils am Umklammerungsring erfolgen.

Der erfindungsgemäße Beschlag ist vielseitig einsetzbar, beispielsweise zur Anlenkung der Lehne eines Fahrzeugsitzes, zur Neigungseinstellung einer Oberschenkelunterstützung oder zum sonstigen Klappen und Verriegeln eines Teiles des Fahrzeugsitzes. Dabei kann der Beschlag ein Rastbeschlag sein, wie beispielsweise in der DE 101 05 282 A1 beschrieben, oder als Getriebebeschlag ausgebildet sein, wie beispielsweise in der DE 101 20 854 C1 mit zwei Hohlrädern und einem zentralen Planetenrad beschrieben.

Im Folgenden ist die Erfindung anhand zweier in der Zeichnung dargestellter Ausführungsbeispiele näher erläutert. Es zeigen
- Fig. 1: eine dreiteilige Darstellung des ersten Ausführungsbeispiels, welche - von rechts nach links dargestelle - drei Montageschritte zur Montage eines Beschlags gemäß dem Ausführungsbeispiel zeigt, wobei im ersten Schritt nur ein Umklammerungsring und ein Zwischenring dargestellt sind,
- Fig. 2: einen Schnitt quer durch einen Beschlag während des Zusammenbaus,
- Fig. 3: eine Detailansicht des Details III von Fig. 2,
- Fig. 4: eine weitere perspektivische Darstellung des zweiten Zusammenbauschritts von Fig. 1,
- Fig. 5: einen Schnitt durch den äußeren Bereich des Beschlags nach vollständigem Zusammenbau,
- Fig. 6: eine schematische Darstellung eines Fahrzeugsitzes mit erfindungsgemäßen Beschlägen,
- Fig. 7: eine Detailansicht des Außenbereichs des Beschlags,
- Fig. 8: einen Schnitt entlang Linie VIII-VIII in Fig. 7,
- Fig. 9: einen Schnitt entlang Linie IX-IX in Fig. 1,
- Fig. 10: einen Schnitt durch den Lagerbereich gemäß dem zweiten Ausführungsbeispiel, und
- Fig. 11: einen Schnitt durch einen Beschlag gemäß dem Stand der Technik.

Im ersten Ausführungsbeispiel weist ein als Rastbeschlag ausgebildeter Beschlag 1 ein näherungsweise scheibenförmiges erstes Beschlagteil 11 und ein ebenfalls näherungsweise scheibenförmiges zweites Beschlagteil 12 auf, die relativ zueinander um eine zentrale Achse A drehbar sind. Die nachfolgenden Richtungsangaben beziehen sich auf das Zylinderkoordinatensystem, welches durch diese zentrale Achse A definiert ist.

Wie in der DE 101 05 282 A1 beschrieben, sind am ersten Beschlagteil 11 Führungssegmente ausgebildet, die zwischen sich wenigstens einen, vorliegend zwei radial bewegliche, gezahnte Riegel führen, welche mit einem Zahnkranz des als Hohlrad ausgebildeten zweiten Beschlagteils 12 zusammenwirken, um den Beschlag 1 zu verriegeln.

Ein im Querschnitt im Wesentlichen L-förmiger Umklammerungsring 21 weist einerseits einen in Umfangsrichtung verlaufenden, im Wesentlichen hohlzylindrischen Bereich 22 auf, mit welchem er auf der radial nach außen weisenden Umfangsfläche des ersten Beschlagteils 11 sitzt und mit diesem fest verbunden ist. Andererseits weist der Umklammerungsring 21 auf seiner vom ersten Beschlagteil 11 abgewandten Stirnseite einen Rand 23 auf, welcher unter Bildung einer ringförmigen Stirnfläche das zweite Beschlagteil 12 übergreift und in axialer Richtung sichert. In radialer Richtung ist zwischen dem zweiten Beschlagteil 12 und dem Umklammerungsring 21 ein Spiel vorgesehen, um eine ungehinderte Drehbewegung des zweiten Beschlagteils 12 zu ermöglichen.

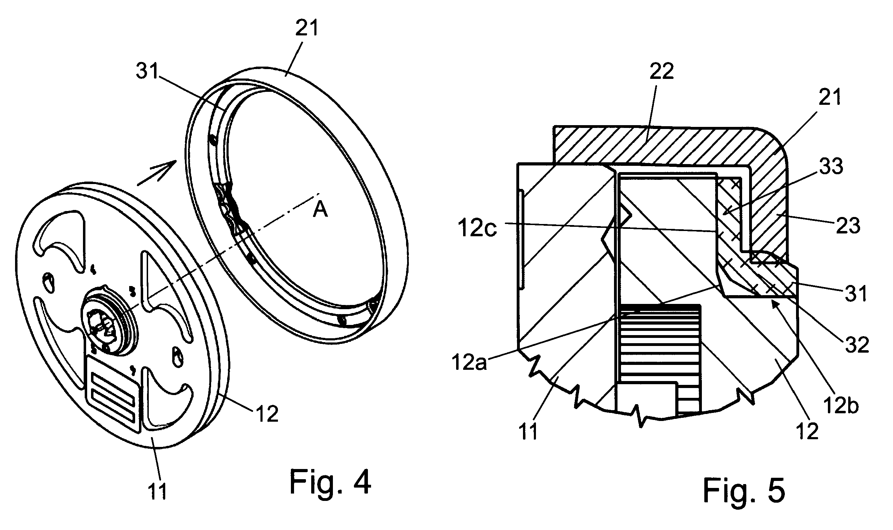

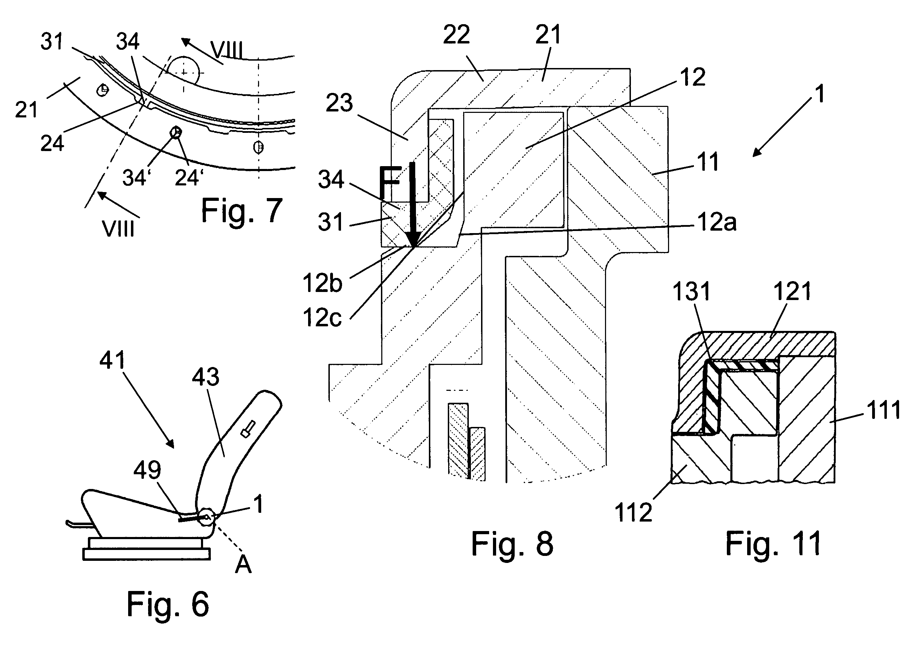

In dem Bereich zwischen dem zweiten Beschlagteil 12 und dem Umklammerungsring 21 ist ein auf einem radial nicht ganz außen liegenden Absatz 12a (gebildet durch eine zylinderförmige Lagerfläche 12b und eine sich im wesentlichen in radialer Richtung nach außen erstreckende Stirnfläche 12c) auf einer Lagerfläche 12b des zweiten Beschlagteils 12 sitzender, im Querschnitt im Wesentlichen L-förmiger Zwischenring 31 aus Kunststoff angeordnet, welcher in umgekehrter Anordnung zum Umklammerungsring 21 einen inneren, im Wesentlichen hohlzylindrischen Bereich 32 und einen daran anschließenden, sich nach außen erstreckenden, ringförmigen Randbereich 33 aufweist. Der quer zur axialen Richtung verlaufenden Randbereich 33 des Zwischenrings 31 ist somit zwischen dem Rand 23 des Umklammerungsrings 21 und dem zweiten Beschlagteil 12 positioniert. Der hohlzylindrische Bereich 32 des Zwischenrings 31 ist an der im Wesentlichen zylinderförmigen Innenumfangsfläche des ringförmigen Randes 23 des Umklammerungsrings 21 positioniert und auf einer im Wesentlichen zylinderförmigen Außenmantelfläche des Absatzes 12a des zweiten Beschlagteils 12, welche besagte Lagerfläche 12b bildet, drehbar positioniert, so dass dieser Bereich das Lager zwischen der Anordnung erstes Beschlagteil 11 + Umklammerungsring 21 + Zwischenring 31 und dem zweiten Beschlagteil 12 bildet, sprich das erste Beschlagteil 11 am zweiten Beschlagteil 12 lagert. Im Prinzip kann der Zwischenring 31 auch entfallen (vgl. zweites Ausführungsbeispiel), so dass das erste Beschlagteil 11 über den fest hiermit verbundenen Umklammerungsring 21 direkt am zweiten Beschlagteil 12 gelagert ist. Gemäß der Darstellung von Fig. 5 liegt der Zwischenring 31 mit seiner zum zweiten Beschlagteil 12 weisenden Seitenfläche an dieser in radialer Richtung verlaufenden Fläche des Absatzes 12a an, jedoch kann in diesem Bereich auch ein Abstand vorgesehen sein, wie in Fig. 8 dargestellt. Hierbei ist zu Fig. 5 anzumerken, dass die elastische/plastische Verformung des Zwischenrings 31, die sich während der Montage des Beschlags 1 ergibt, nicht dargestellt ist, sondern eine Überschneidung des Zwischenrings 31 und des Umklammerungsrings 21 im Bereich der Nocken 34 dargestellt ist. Fig. 8 zeigt hingegen den verformten Zwischenring 31.

Der Zwischenring 31 weist mindestens drei, vorliegend sechs Nocken 34 auf, die sich in radialer Richtung nach außen in entsprechend tiefe, jedoch in Umfangsrichtung größer ausgebildete Aufnahmen 24 im Umklammerungsring 21 erstrecken (siehe Fig. 7). Ferner weist der Zwischenring 31 Zapfen 34' auf, die sich in axialer Richtung vom Randbereich 33 des Zwischenrings 31 in Richtung Umklammerungsring 24 erstrecken und in entsprechenden Öffnungen 24' des Umklammerungsrings 21 aufgenommen sind. Diese Anordnung stellt eine drehfeste Mitnahme des Zwischenrings 31 durch den Umklammerungsring 21 sicher. Die Ausgestaltung der drehfesten Mitnahme kann jedoch auch auf beliebige andere geeignete Weise erfolgen, insbesondere können beispielsweise nur die Nocken 34 in Verbindung mit den Aufnahmen 24 oder nur die Zapfen 34' in Verbindung mit den Öffnungen 24' des Umklammerungsrings 21 vorgesehen sein.

Am Zwischenring 31 sind ferner drei Federbereiche, 35 ausgebildet, welche aus dem hohlzylindrischen Bereich 32 sich in radialer Richtung nach außen erstrecken und auf der Innenseite des Umklammerungsrings 21 anliegen. Dadurch werden etwaige Toleranzen, beispielsweise Durchmessertoleranzen aufgrund von Wasseraufnahme des Zwischenrings 31, ausgeglichen, ohne dass die Relativdrehung zwischen dem ersten und zweiten Beschlagteil behindert wird. Zusätzlich wirken die Nocken 34 dämpfend.

Der Zwischenring 31 ist mit mehreren Fasen ausgebildet, wobei eine am Außenumfang vorgesehene erste Fase beim Einlegen des Zwischenrings 31 in den Umklammerungsring 21 hilft, d.h. als Einführschräge dient. Am Innenumfang ist auf der Seite des Randbereichs 33 eine abgestufte Fase ausgebildet, wobei der randseitige Bereich eine Freimachung zur Anpassung an die Gestalt des Absatzes 12a in diesem Bereich ist, der Bereich benachbart der Innenmantelfläche dient als Einführschräge zum Aufpassen auf das zweite Beschlagteil 12 und verhindert eine Beschädigung der Innenmantelfläche, welche bei Benutzung an der Lagerfläche 12b des zweiten Beschlagteils 12 drehbar gelagert ist.

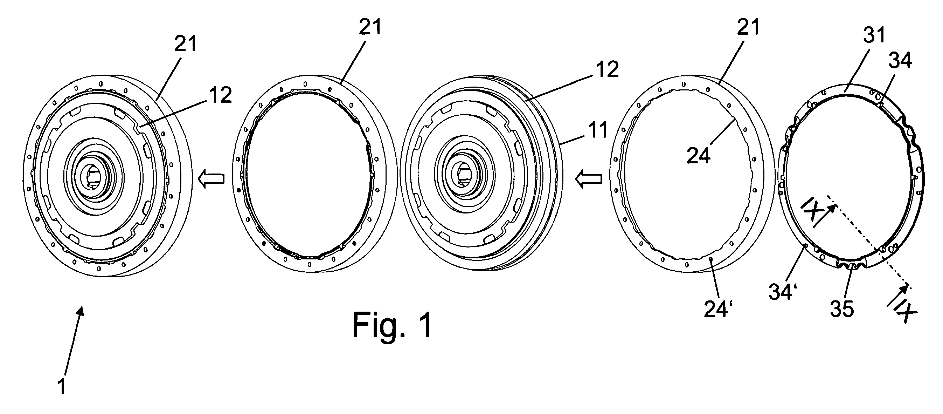

Die Montage des Beschlags 1 erfolgt auf folgende Weise: in einem ersten Schritt werden - unabhängig voneinander - die Beschlagteile 11 und 12 sowie die weiteren, nicht näher beschriebenen Elemente, die zwischen den beiden Beschlagteilen 11 und 12 angeordnet sind, vormontiert, und getrennt hiervon der Zwischenring 31 in den Umklammerungsring 21 eingelegt, wie in Fig. 1 rechts dargestellt. Anschließend wird die Baugruppe Zwischenring-Umklammerungsring auf die Beschlagteilbaugruppe gesetzt und die beiden Baugruppen zusammengepresst.

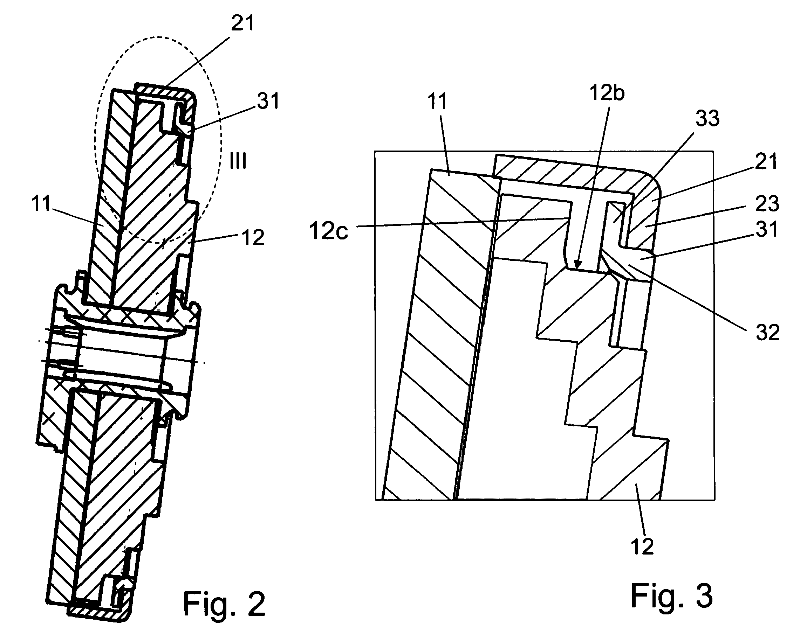

Hierbei weist der Zwischenring 31 vor der Montage im Bereich der Nocken 34 ein leichtes Übermaß auf. Um das Zusammenpressen zu vereinfachen, weisen der Absatz 12a und der entsprechende Bereich des Zwischenrings 31 eine Fase (oder Abrundung) auf, wie aus Fig. 8 ersichtlich. Entsprechendes ist auch aus der Darstellung von Fig. 3, welche einen leicht modifizierten Zwischenring 31 zeigt, ersichtlich. Eine weitere Abschrägung oder Fase ist an der Außenkante des Zwischenrings 31 wie auch der entsprechenden Innenkante des Absatzes 12a vorgesehen.

Durch das Zusammenpressen verringert sich die Höhe der Nocken 34, wobei sich der Zwischenring 31 während der Montage unter elastischer und/oder plastischer Verformung an die Geometrie des Umklammerungsrings 21 anpasst. Aufgrund der Nocken 34 in Verbindung mit den Aufnahmen 24 und der Zapfen 34' in Verbindung mit den Öffnungen 24' ist der Zwischenring 31 drehfest im Umklammerungsring 21 positioniert. Ferner wirkt eine gewisse Vorspannkraft F, wie in Fig. 8 dargestellt, zwischen dem Zwischenring 31 und dem zweiten Beschlagteil 12, d.h. das zweite Beschlagteil 12 ist unter radialer Vorspannung gelagert.

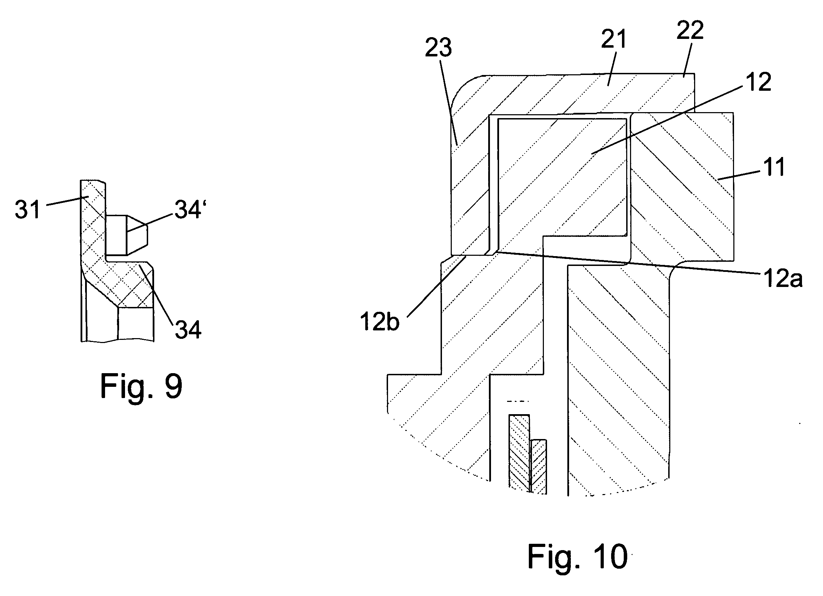

Gemäß dem in Fig. 10 schematisch dargestellten zweiten Ausführungsbeispiel entfällt der Zwischenring. Hierbei ist der Zwischenring 21 entsprechend dem ersten Ausführungsbeispiel in seinem hohlzylindrischen Bereich 22 fest mit dem ersten Beschlagteil 11 verbunden. Im Unterschied zum ersten Ausführungsbeispiel ist der Rand 23 weiter in radialer Richtung nach innen gezogen, so dass die Innenmantelfläche des Randes 23 direkt in Anlage an die Lagerfläche 12b des Absatzes 12a des zweiten Beschlagteils 12 ist. Ansonsten entspricht das zweite Ausführungsbeispiel dem ersten Ausführungsbeispiel, so dass hierauf nicht näher eingegangen wird.

Gemäß einer nicht in der Zeichnung dargestellen Variante des zweiten Ausführungsbeispiels ist der Endbereich des Umklammerungsrings 21, in welchem das zweite Beschlagteil 12 gelagert ist, mit einer reibungsvermindernden Beschichtung versehen.

Zur Erhöhung der Sicherheit, insbesondere der Crashsicherheit, ist der Außenumfang des zweiten Beschlagteils 12 derart bemessen, dass sich die Außenmantelfläche bei großen Belastungen zusätzlich am Umklammerungsring 21 direkt abstützen kann, insbesondere ohne dass der Zwischenring 31 dazwischen angeordnet ist. Im normalen Betrieb befindet sich jedoch ein Spalt zwischen den beiden Teilen, wobei dieser Spalt mit Fett gefüllt sein kann, um eine dämpfende Schicht zur Verfügung zu stellen und im (unerwünschten) Falle eines radialen Kontakts Geräusche zu verhindern.

Der erfindungsgemäße Beschlag 1 kann bei einem Fahrzeugsitz 41 für ein Kraftfahrzeug verwendet werden, beispielsweise für die Neigungseinstellung, Tischklappung oder anderweitigen Anlenkung von dessen Lehne 43. Dabei sitzt in der Regel ein manuell betätigbarer Handhebel 49 auf einer mit der zentralen Achse A fluchtenden Übertragungsstange zwischen zwei erfindungsgemäßen Beschlägen 1 und sorgt für ein synchrones Entriegeln derselben.

### Bezugszeichenliste

- 1: Beschlag
- 11: erstes Beschlagteil
- 12: zweites Beschlagteil
- 12a: Absatz
- 12b: Lagerfläche
- 12c: Stirnfläche
- 21: Umklammerungsring
- 22: hohlzylindrischer Bereich
- 23: Rand
- 24: Aussparung
- 24': Öffnung
- 31: Zwischenring
- 32: hohlzylindrischer Bereich
- 33: Randbereich
- 34: Nocken
- 34': Zapfen
- 35: Federbereich
- 41: Fahrzeugsitz
- 43: Lehne
- 49: Handhebel
- A: zentrale Achse
- F: radiale Vorspannkraft

### Stand der Technik

- 111: erstes Beschlagteil
- 112: zweites Beschlagteil
- 121: Umklammerungsring
- 131: Zwischenring

## Patentansprüche

1. Beschlag (1) für einen Fahrzeugsitz (41), insbesondere für einen Kraftfahrzeugsitz, mit
einem ersten Beschlagteil (11),
einem relativ zu diesem um eine zentrale Achse (A) drehbaren zweiten Beschlagteil (12), und
einem im Querschnitt im Wesentlichen L-förmigen Umklammerungsring (21), wobei der Umklammerungsring (21) einen hohlzylindrischen Bereich (22), mit welchem er auf einer radial nach außen weisenden Umfangsfläche des ersten Beschlagteils (11) sitzt und mit diesem ersten Beschlagteil (11) fest verbunden ist, und - auf seiner vom ersten Beschlagteil (11) abgewandten Stirnseite - einen in radialer Richtung nach innen gezogenen Rand (23) aufweist, welcher unter Bildung einer ringförmigen Stirnfläche das zweite Beschlagteil (12) übergreift und es relativ zum ersten Beschlagteil (11) drehbar am zweiten Beschlagteil (12) sichert,
**dadurch gekennzeichnet, dass** am zweiten Beschlagteil (12) ein gegenüber dem maximalen Außendurchmesser radial nach innen versetzter Absatz (12a) mit sich in radialer Richtung nach außen erstreckender Stirnfläche (12c) und einer zylinderförmigen Lagerfläche (12b) ausgebildet ist, wobei die Lagerfläche (12b) einen Durchmesser hat, der kleiner als der maximale Außendurchmesser des zweiten Beschlagteils (12) ist, und die zylinderförmige Lagerfläche (12b) den Rand (23) des Umklammerungsrings (21) direkt oder indirekt lagert.

2. Beschlag nach Anspruch 1, **dadurch gekennzeichnet, dass** ein Zwischenring (31) vorgesehen ist, der zwischen dem Umklammerungsring (21) und dem zweiten Beschlagteil (12) angeordnet ist, wobei der Zwischenring (31) zwischen dem Rand (23) des Umklammerungsrings (21) und dem Absatz (12a) des zweiten Beschlagteils (12) angeordnet ist.

3. Beschlag nach Anspruch 2, **dadurch gekennzeichnet, dass** der Zwischenring (31) einen hohlzylindrischen Bereich (32) und einen Randbereich (33) aufweist, wobei das erste Beschlagteil (11) am hohlzylindrischen Bereich (32) des Zwischenrings (31) an dessen Innenmantelfläche gelagert ist.

4. Beschlag nach einem der Ansprüche 2 bis 3, **dadurch gekennzeichnet, dass** der Umklammerungsring (21) Mittel zum drehfesten Halten des Zwischenrings (31) aufweist.

5. Beschlag nach Anspruch 4, **dadurch gekennzeichnet, dass** der Zwischenring (31) Nocken (34) und/oder Zapfen (34') aufweist, welche mit entsprechenden Aussparungen (24) oder Öffnungen (24') des Umklammerungsrings (21) zusammenwirken.

6. Beschlag nach einem der Ansprüche 2 bis 5, **dadurch gekennzeichnet, dass** das zweite Beschlagteil (12) und der Zwischenring (31) mit Fasen versehen sind, um das Aufschieben des Zwischenrings (31) auf den Absatz (12a) des zweiten Beschlagteils (12) zu erleichtern.

7. Beschlag nach einem der Ansprüche 2 bis 6, **dadurch gekennzeichnet, dass** der Zwischenring (31) eine radiale Vorspannkraft (F) auf das zweite Beschlagteil (12) ausübt.

8. Beschlag nach einem der vorhergehenden Ansprüche, **dadurch gekennzeichnet, dass** bei ordnungsgemäßem Gebrauch des Beschlags (1) ein Spalt zwischen dem Außenumfang des zweiten Beschlagteils (12) und dem Umklammerungsring (21) vorgesehen ist, und dass im Crashfall eine zusätzliche direkte Abstützung des zweiten Beschlagteils (12) am Umklammerungsring (21) möglich ist.

9. Beschlag nach Anspruch 8, **dadurch gekennzeichnet, dass** in dem Spalt ein Fett aufgenommen ist.

10. Beschlag nach einem der Ansprüche 3 bis 9, **dadurch gekennzeichnet, dass** am Zwischenring (31) mindestens ein Federbereich (35) ausgebildet ist, welcher sich aus dem hohlzylindrischen Bereich (32) in radialer Richtung nach außen erstreckt und auf der Innenseite des Umklammerungsrings (21) anliegt.

11. Beschlag nach Anspruch 10, **dadurch gekennzeichnet, dass** am Zwischenring (31) genau drei Federbereiche (35) ausgebildet sind.

12. Beschlag nach Anspruch 1, **dadurch gekennzeichnet, dass** die Innenmantelfläche des Randes (23) direkt in Anlage an die Lagerfläche (12b) des Absatzes (12a) des zweiten Beschlagteils (12) ist.

13. Beschlag nach Anspruch 12, **dadurch gekennzeichnet, dass** der Endbereich des Umklammerungsrings (21), in welchem das zweite Beschlagteil (12) gelagert ist, mit einer reibungsvermindernden Beschichtung versehen ist.

14. Fahrzeugsitz (41) mit einer Lehne (43), **gekennzeichnet durch** einen Beschlag (1) nach einem der Ansprüche 1 bis 13 zur Anlenkung der Lehne (43).

## Claims

1. Fitting (1) for a vehicle seat (41), in particular for a motor vehicle seat, having
a first fitting member (11),
a second fitting member (12) which is rotatable relative thereto about a center axis (A) and
a clamping ring (21) which is substantially L-shaped in cross section, wherein the clamping ring (21) has a hollow-cylindrical region (22), with which it is located on a radially outwardly directed peripheral face of the first fitting member (11) and is securely connected to that first fitting member (11), and - at the end face thereof facing away from the first fitting member (11) - an edge (23) which is drawn inwards in a radial direction and which engages over the second fitting member (12) so as to form an annular end face and rotatably secures it to the second fitting member (12) relative to the first fitting member (11),
**characterized in that** a shoulder (12a) which is offset radially inwards relative to the maximum outer diameter and which has an end face (12c) which extends outwards in a radial direction and a cylindrical bearing face (12b) is constructed on the second fitting member (12), with the bearing face (12b) having a diameter which is smaller than the maximum outer diameter of the second fitting member (12), and the cylindrical bearing face (12b) supporting the edge (23) of the clamping ring (21) directly or indirectly.

2. Fitting according to Claim 1, **characterized in that** there is provided an intermediate ring (31) which is arranged between the clamping ring (21) and the second fitting member (12), with the intermediate ring (31) being arranged between the edge (23) of the clamping ring (21) and the shoulder (12a) of the second fitting member (12).

3. Fitting according to Claim 2, **characterized in that** the intermediate ring (31) has a hollow-cylindrical region (32) and an edge region (33), with the first fitting member (11) being supported on the hollow-cylindrical region (32) of the intermediate ring (31) on the inner surface thereof.

4. Fitting according to either Claim 2 or Claim 3, **characterized in that** the clamping ring (21) has means for securing the intermediate ring (31) in a rotationally secure manner.

5. Fitting according to Claim 4, **characterized in that** the intermediate ring (31) has cams (34) and/or studs (34') which cooperate with corresponding recesses (24) or openings (24') of the clamping ring (21).

6. Fitting according to one of Claims 2 to 5, **characterized in that** the second fitting member (12) and the intermediate ring (31) are provided with chamfers in order to make it easier to push the intermediate ring (31) onto the shoulder (12a) of the second fitting member (12).

7. Fitting according to one of Claims 2 to 6, **characterized in that** the intermediate ring (31) applies a radial pretensioning force (F) to the second fitting member (12).

8. Fitting according to one of the preceding claims, **characterized in that**, during correct use of the fitting (1), a gap is provided between the outer periphery of the second fitting member (12) and the clamping ring (21), and **in that**, in the event of a crash, additional direct support of the second fitting member (12) on the clamping ring (21) is possible.

9. Fitting according to Claim 8, **characterized in that** a lubricant is received in the gap.

10. Fitting according to one of Claims 3 to 9, **characterized in that** there is constructed on the intermediate ring (31) at least one resilient region (35) which extends out of the hollow-cylindrical region (32) outwards in a radial direction and adjoin(s) the inner side of the clamping ring (21).

11. Fitting according to Claim 10, **characterized in that** precisely three resilient regions (35) are constructed on the intermediate ring (31).

12. Fitting according to Claim 1, **characterized in that** the inner surface of the edge (23) is in direct abutment with the bearing face (12b) of the shoulder (12a) of the second fitting member (12).

13. Fitting according to Claim 12, **characterized in that** the end region of the clamping ring (21) in which the second fitting member (12) is supported is provided with a friction-reducing coating.

14. Vehicle seat (41) having a backrest (43), **characterized by** a fitting (1) according to one of Claims 1 to 13 for coupling the backrest (43).

## Revendications

1. Ferrure (1) pour un siège de véhicule (41), en particulier pour un siège de véhicule à moteur, avec une première partie de ferrure (11),
une deuxième partie de ferrure (12) pouvant tourner par rapport à celle-ci autour d'un axe central (A), et
une bague de serrage (21) essentiellement en forme de L en section transversale,
dans laquelle la bague de serrage (21) présente une région cylindrique creuse (22), avec laquelle elle prend appui sur une face périphérique tournée radialement vers l'extérieur de la première partie de ferrure (11) et elle est assemblée fixement à cette première partie de ferrure (11), et - sur un côté frontal situé à l'opposé de la première partie de ferrure (11) - un bord (23) tiré vers l'intérieur en direction radiale, qui recouvre la deuxième partie de ferrure (12) en formant une face frontale annulaire et la bloque sur la deuxième partie de ferrure (12) de façon rotative par rapport à la première partie de ferrure (11),
**caractérisée en ce qu'**un épaulement (12a) déporté radialement vers l'intérieur par rapport au diamètre extérieur maximal est formé sur la deuxième partie de ferrure (12), avec une face frontale (12c) s'étendant vers l'extérieur en direction radiale et une face d'appui cylindrique (12b), dans laquelle la face d'appui (12b) présente un diamètre qui est plus petit que le diamètre extérieur maximal de la deuxième partie de ferrure (12), et la face d'appui cylindrique (12b) supporte directement ou indirectement le bord (23) de la bague de serrage (21).

2. Ferrure selon la revendication 1, **caractérisée en ce qu'**il est prévu une bague intermédiaire (31), qui est disposée entre la bague de serrage (21) et la deuxième partie de ferrure (12), dans laquelle la bague intermédiaire (31) est disposée entre le bord (23) de la bague de serrage (21) et l'épaulement (12a) de la deuxième partie de ferrure (12).

3. Ferrure selon la revendication 2, **caractérisée en ce que** la bague intermédiaire (31) présente une région cylindrique creuse (32) et une région de bord (33), dans laquelle la première partie de ferrure (11) est supportée sur la région cylindrique creuse (32) de la bague intermédiaire (31) sur la surface latérale intérieure de celle-ci.

4. Ferrure selon l'une des revendications 2 à 3, **caractérisée en ce que** la bague de serrage (21) présente des moyens pour le blocage sans rotation de la bague intermédiaire (31).

5. Ferrure selon la revendication 4, **caractérisée en ce que** la bague intermédiaire (31) présente des cames (34) et/ou des tenons (34'), qui coopèrent avec des découpes (24) ou des ouvertures (24') correspondantes de la bague de serrage (21).

6. Ferrure selon l'une quelconque des revendications 2 à 5, **caractérisée en ce que** la deuxième partie de ferrure (12) et la bague intermédiaire (31) sont dotées de chanfreins, destinés à faciliter le glissement de la bague intermédiaire (31) sur l'épaulement (12a) de la deuxième partie de ferrure (12).

7. Ferrure selon l'une quelconque des revendications 2 à 6, **caractérisée en ce que** la bague intermédiaire (31) exerce une force de précontrainte radiale (F) sur la deuxième partie de ferrure (12).

8. Ferrure selon l'une quelconque des revendications précédentes, **caractérisée en ce que**, en utilisation normale de la ferrure (1), il est prévu une fente entre la périphérie extérieure de la deuxième partie de ferrure (12) et la bague de serrage (21), et **en ce qu'**en cas de collision un appui direct supplémentaire de la deuxième partie de ferrure (12) sur la bague de serrage (21) est possible.

9. Ferrure selon la revendication 8, **caractérisée en ce qu'**une graisse est logée dans la fente.

10. Ferrure selon l'une quelconque des revendications 3 à 9, **caractérisée en ce qu'**au moins une région de ressort (35) est formée sur la bague intermédiaire (31), laquelle s'étend en direction radiale vers l'extérieur hors de la région cylindrique creuse (32) et repose sur le côté intérieur de la bague de serrage (21).

11. Ferrure selon la revendication 10, **caractérisée en ce qu'**exactement trois régions de ressort (35) sont formées sur la bague intermédiaire (31).

12. Ferrure selon la revendication 1, **caractérisée en ce que** la surface latérale intérieure du bord (23) est directement en appui sur la face d'appui (12b) de l'épaulement (12a) de la deuxième partie de ferrure (12).

13. Ferrure selon la revendication 12, **caractérisée en ce que** la région d'extrémité de la bague de serrage (21), dans laquelle la deuxième partie de ferrure (12) est appuyée, est munie d'un revêtement réduisant le frottement.

14. Siège de véhicule (41) avec un dossier (43), **caractérisé par** une ferrure (1) selon l'une quelconque des revendications 1 à 13 pour l'articulation du dossier (43).
